# EUROPEAN PATENT APPLICATION

(11) **EP 1 964 556 A1**
(43) Date of publication of application: **03.09.2008**
(21) Application number: 06835012.3
(22) Date of filing: 20.12.2006
(51) Int. Cl.: A61K 31/216, A61K 31/19, A61K 38/49, A61P 7/02, C07C 233/49

(54) **THERAPEUTIC AGENT OR THERAPEUTIC METHOD FOR ISCHEMIC STROKE**

(30) Priority: 21.12.2005 US 752054 P; 14.07.2006 US 830686 P
(71) Applicant: SHIONOGI & CO., LTD., Osaka-shi, Osaka 541-0045 (JP)
(72) Inventor: NINOMIYA, Mitsuyoshi, Osaka 553-0002 (JP); NAGAFUJI, Toshiaki, Osaka 553-0002 (JP)
(74) Representative: Hayes, Adrian Chetwynd
(86) International application number: PCT/JP2006/325342
(87) International publication number: WO 2007/072845

(57) **Abstract**

A drug for alleviating bleeding tendency caused by a thrombus removing means comprising, as an active ingredient, a compound represented by formula (I): 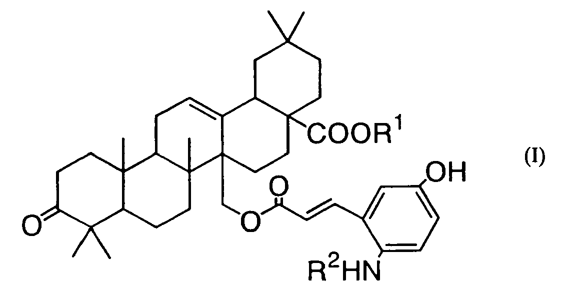 (wherein R¹ represents hydrogen or a metabolic ester residue, R² represents hydrogen or -R³-R⁴ (wherein R³ represents -SO₃-, -CH₂COO-, -COCOO- or -COR⁵COO- (wherein R⁵ represents lower alkylene or lower alkenylene), and R⁴ represents hydrogen or a metabolic ether residue)),
or a pharmaceutically acceptable salt thereof, or a solvate thereof.

## Description

### [Technical field]

The present invention relates to a remedy for, and a method of treating ischemic cerebral stroke, and a drug for alleviating bleeding tendency caused by a thrombus removing means.

### [Background art]

Ischemic cerebral stroke such as cerebral infarction was once thought that, when developed once, curing is difficult, but it was confirmed that administration of tissue plasminogen activator (hereinafter, tPA) within 3 hours after stroke onset is effective, and tPA was approved as a remedy for ultraacute phase cerebral infarction by US Food and Drug Administration (FDA) in 1995.

In Japan, since 1991, recombinant tPA has been sold for indication of "coronary thrombolysis in acute myocardial infarction (within 6 hours after onset)," and was additionally approved regarding "improvement in functional disorder accompanied with ischemic cerebral vascular disorder acute phase (within 3 hours after stroke onset)" in October, 2005.

tPA has high affinity for fibrin, and fibrinogenolysis accompanied with plasminogen activation in circulating blood hardly occurs. Therefore, it is said that increase in bleeding tendency which is the side effect is little, but increase in bleeding tendency by tPA administration can not be avoided and, upon use thereof, severe application criteria is determined.

In addition, an apparatus for directly destructing or removing a thrombus using a catheter was variously studies and developed, and Merci (registered trademark) Retrieval System has been already approved by FDA (Non-Patent Literature 1). Since when this mechanical thrombus isolating apparatus is used, the blood reflow is possible in a shorter time as compared with thrombolysis due to a drug, and it is possible to remove a great thrombus which is difficult to be lysed with a drug, this is a currently expected treating method. However, although the blood reflow effect has been confirmed also within 8 hours from stroke onset with this apparatus, its intracerebral hemorrhage risk is still high like tPA, and a safer and effective new treating method is desired.

A compound represented by the formula (I): 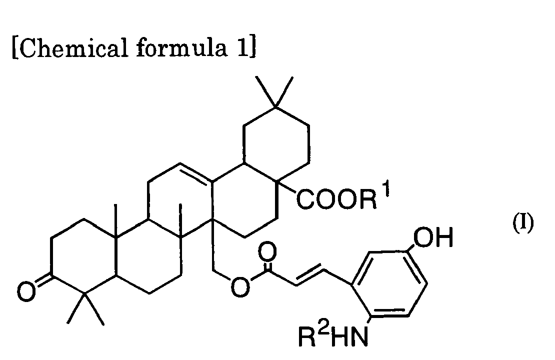 (Wherein R¹ represents hydrogen or a metabolic ester residue, R² represents hydrogen or -R³-R⁴ (wherein R³ represents -SO₃-, -CH₂COO-, -COCOO- or -COR⁵COO- (wherein R⁵ represents lower alkylene or lower alkenylene), and R⁴ represents hydrogen or a metabolic ester residue)) or a pharmaceutically acceptable salt or a solvate thereof (hereinafter, referred to as compound (I)), which is used in the present invention, is the known compound described in Patent Literature 1, and it is described that it has endothelin antagonism.

A method of treating cerebral infarction with a combination of tPA and other drug is described in Non-Patent Literatures 2 and 3, but a combination of the compound (I) and tPA is not described or suggested in both of Literatures.

Non-Patent Literature 4 shows that, by administration of SUN9216 (modified tPA) alone, the activity of inhibiting development of a cerebral infarction lesion is recognized, but when FR139317 which is the endothelin antagonist is used together, this effect disappears.
[Patent Literature 1] Japanese Patent Application Laid Open (JP-A) No.7-53484
[Non-Patent Literature 1] American Journal of Neuroradiol, 2004 vol.25, p.1812-1815
[Non-Patent Literature 2] Stroke, 2001, vol.32, p.2635-2640
[Non-Patent Literature 3] Brain Research, 2003, vol.959, p.169-172
[Non-Patent Literature 4] European Journal of Pharmacology, 1995, vol.275, No.1, p.17-21

### [Disclosure of the invention]

### [Problems to be solved by the invention]

A remedy for ischemic cerebral stroke (cerebral infarction etc.) by combining the compound (I) and a thrombus removing means (preferably, tPA analogue or thrombus removing device) is provided.

### [Means to solve the problems]

The present invention provides the following:
(1) A drug for alleviating bleeding tendency caused by a thrombus removing means comprising, as an active ingredient, a compound represented by formula (I): 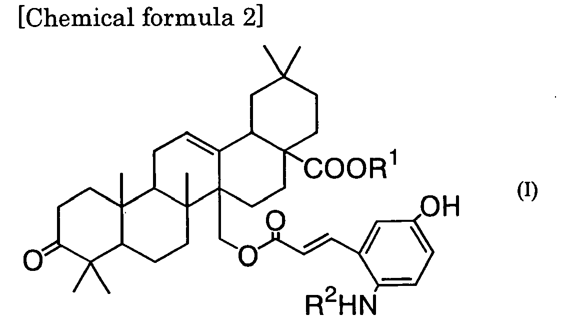 (wherein R¹ represents hydrogen or a metabolic ester residue, R² represents hydrogen or -R³-R⁴ (wherein R³ represents -SO₃-, -CH₂COO-, -COCOO- or -COR⁵COO- (wherein R⁵ represents lower alkylene or lower alkenylene), and R⁴ represents hydrogen or a metabolic ether residue)),
   or a pharmaceutically acceptable salt thereof, or a solvate thereof.
(2) The drug for alleviating bleeding tendency according to (1), wherein the thrombus removing means is a tissue plasminogen activator analogue and/or a thrombus removing device.
   (2-1) The drug for alleviating bleeding tendency according to (1) or (2), wherein administration of the compound represented by the formula (1) as defined in (1), or a pharmaceutically acceptable salt thereof, or a solvate thereof is initiated immediately after or within 20 hours from ischemic cerebral stroke onset.
   (2-2) The drug for alleviating bleeding tendency according to any one of (1) to (2-1), wherein application of the thrombus removing means is initiated during administration of the compound represented by the formula (I) as defined in (1), or a pharmaceutically acceptable salt thereof, or a solvate thereof, or within 5 hours after administration completion.
   (2-3) The drug for alleviating bleeding tendency according to any one of (1) to (2-2), wherein the compound represented by the formula (I) as defined in (1), or a pharmaceutically acceptable salt thereof, or a solvate thereof is administered by an administration route selected from the group consisting of intravenous injection, arterial injection, subcutaneous injection, intracerebral administration and intraspinal administration.
   (2-4) The drug for alleviating bleeding tendency according to any one of (1) to (2-3), wherein the compound represented by the formula (I) as defined in (1), or a pharmaceutically acceptable salt thereof, or a solvate thereof is administered by drip infusion.
   (2-5) The drug for alleviating bleeding tendency according to any one of (1) to (2-4), wherein the compound represented by the formula (I) as defined in (1), or a pharmaceutically acceptable salt thereof, or a solvate thereof is administered at an amount of 1 to 500 mg/kg.
   (2') A drug for alleviating bleeding tendency caused by a tissue plasminogen activator analogue, comprising the compound represented by the formula (I) as defined in (1), or a pharmaceutically acceptable salt thereof, or a solvate thereof as an active ingredient.
   (2'-1) The drug for alleviating bleeding tendency caused by a tissue plasminogen activator analogue according to (2'), wherein administration of the compound represented by the formula (I) as defined in (1), or a pharmaceutically acceptable salt thereof, or a solvate thereof is initiated immediately after, to within 20 hours after ischemic cerebral stroke onset.
   (2'-2) The drug for alleviating bleeding tendency caused by a tissue plasminogen activator analogue according to (2') or (2'-1), wherein administration of the tissue plasminogen activator analogue is initiated during administration of, or within 5 hours after completion of administration of the compound represented by the formula (I) as defined in (1), or a pharmaceutically acceptable salt thereof, or a solvate thereof.
   (2'-3) The drug for alleviating bleeding tendency caused by a tissue plasminogen activator analogue according to any one of (2') to (2'-2), wherein the compound represented by the formula (I) as defined in (1), or a pharmaceutically acceptable salt thereof, or a solvate thereof is administered by administration route selected from the group consisting of intravenous injection, arterial injection, subcutaneous injection, intracerebral administration, and intraspinal administration.
   (2'-4) The drug for alleviating bleeding tendency caused by a tissue plasminogen activator analogue according to any one of (2') to (2'-3), wherein the compound represented by the formula (I) as defined in (1), or a pharmaceutically acceptable salt thereof, or a solvate thereof is administered by drip infusion.
   (2'-5) The drug for alleviating bleeding tendency caused by a tissue plasminogen activator analogue according to any one of (2') to (2'-4), wherein the compound represented by the formula (1) as defined in (1), or a pharmaceutically acceptable salt thereof, or a solvate thereof is administered at an amount of 1 to 500 mg/kg.
   (2'-6) A pharmaceutical composition for alleviating bleeding tendency caused by a tissue plasminogen activator, containing the compound represented by the formula (I) as defined in (1), or a pharmaceutically acceptable salt thereof, or a solvate thereof and a tissue plasminogen activator analogue.
(3) A remedy for ischemic cerebral stroke, comprising a combination of the compound represented by the formula (I) as defined in (1), or a pharmaceutically acceptable salt thereof, or a solvate thereof and a tissue plasminogen activator analogue.
(4) The remedy for ischemic cerebral stroke according to (3), which is a kit containing a drug containing the compound represented by the formula (I) as defined in (1), or a pharmaceutically acceptable salt thereof, or a solvate thereof, and a drug containing a tissue plasminogen activator analogue.
(5) The remedy for ischemic cerebral stroke according to (3) or (4), wherein administration of the tissue plasminogen activator analogue is initiated immediately after, to within 20 hours after ischemic cerebral stroke onset.
(6) The remedy for ischemic cerebral stroke according to any one of (3) to (5), wherein administration of the compound represented by the formula (I) as defined in (1), or a pharmaceutically acceptable salt thereof, or a solvate thereof is initiated immediately after, to within 20 hours after ischemic cerebral stroke onset
(7) The remedy for ischemic cerebral stroke according to any one of (3) to (6), wherein administration of the tissue plasminogen activator analogue is initiated during administration of, or within 5 hours after completion of administration of the compound represented by the formula (I) as defined in (1), or a pharmaceutically acceptable salt thereof, or a solvate thereof.
(8) The remedy for ischemic cerebral stroke according to any one of (3) to (7), wherein the compound represented by the formula (I) as defined in (1), or a pharmaceutically acceptable salt thereof, or a solvate thereof is administered by administration route selected from the group consisting of intravenous injection, arterial injection, subcutaneous injection, intracerebral administration and intraspinal administration.
(9) The remedy for ischemic cerebral stroke according to any one of (3) to (8), wherein the compound represented by the formula (I) as defined in (1), or a pharmaceutically acceptable salt thereof, or a solvate thereof is administered by drip infusion.
(10) The remedy for ischemic cerebral stroke according to any one of (3) to (9), wherein the compound represented by the formula (I) as defined in (1), or a pharmaceutically acceptable salt thereof, or a solvate thereof is administered at an amount of 1 to 500 mg/kg.
(11) The remedy for ischemic cerebral stroke according to any one of (3) to (10), wherein the tissue plasminogen activator analogue is administered by administration route selected from the group consisting of intravenous injection, arterial injection, subcutaneous injection, intracerebral administration and intraspinal administration.
(12) The remedy for ischemic cerebral stroke according to any one of (3) to (11), wherein 5% to 20% of a total administration amount of the tissue plasminogen activator analogue is rapidly administered and, thereafter, a remaining amount of the analogue is administered by drip infusion over 20 minutes to 3 hours.
(13) The remedy for ischemic cerebral stroke according to any one of (3) to (12), wherein the tissue plasminogen activator analogue is administered at an amount of 0.3 to 10 mg/kg.
(14) The remedy for ischemic cerebral stroke according to (3), wherein the remedy for ischemic cerebral stroke is a combined drug.
(15) A drug for preventing or improving a motor dysfunction caused by ischemic cerebral stroke, comprising a combination of the compound represented by the formula (I) as defined in (1), or a pharmaceutically acceptable salt thereof, or a solvate thereof and a tissue plasminogen activator analogue.
   (15-1) The drug for preventing or improving a motor dysfunction according to (15), wherein the drug for preventing or improving a motor dysfunction is a kit containing a drug containing the compound represented by the formula (I) as defined in (1), or a pharmaceutically acceptable salt thereof, or a solvate thereof, and a drug containing a tissue plasminogen activator analogue.
   (15-2) The drug for preventing or improving a motor dysfunction according to (15) or (15-1), wherein administration of the tissue plasminogen activator analogue is initiated immediately after, to within 20 hours after ischemic cerebral stroke onset
   (15-3) The drug for preventing or improving a motor dysfunction according to any one of (15) to (15-2), wherein administration of the compound represented by the formula (I) as defined in (1), or a pharmaceutically acceptable salt thereof, or a solvate thereof is initiated immediately after, to within 20 hours after ischemic cerebral stroke onset.
   (15-4) The drug for preventing or improving a motor dysfunction according to any one of (15) to (15-3), wherein administration of the tissue plasminogen activator analogue is initiated during administration of, or within 5 hours after completion of administration of the compound represented by the formula (I) as defined in (1), or a pharmaceutically acceptable salt thereof, or a solvate thereof.
   (15-5) The drug for preventing or improving motor dysfunction according to any one of (15) to (15-4), wherein the compound represented by the formula (I) as defined in (1), or a pharmaceutically acceptable salt thereof, or a solvate thereof is administered by administration route selected from the group consisting of intravenous injection, arterial injection, subcutaneous injection, intracerebral administration and intraspinal administration.
   (15-6) The drug for preventing or improving a motor dysfunction according to any one of (15) to (15-5), wherein the compound represented by the formula (I) as defined in (1), or a pharmaceutically acceptable salt thereof, or a solvate thereof is administered by drip infusion.
   (15-7) The drug for preventing or improving a motor dysfunction according to any one of (15) to (15-6), wherein the compound represented by the formula (I) as defined in (1), or a pharmaceutically acceptable salt thereof, or a solvate thereof is administered at an amount of 1 to 500 mg/kg.
   (15-8) The drug for preventing or improving a motor dysfunction according to any one of (15) to (15-7), wherein the tissue plasminogen activator analogue is administered by administration route selected from the group consisting of intravenous injection, arterial injection, subcutaneous injection, intracerebral administration and intraspinal administration.
   (15-9) The drug for preventing or improving a motor dysfunction according to any one of (15) to (15-8), wherein 5% to 20% of a total administration amount of the tissue plasminogen activator analogue is rapidly administered and, thereafter, a remaining amount of the analogue is administered by drip infusion over 20 minutes to 3 hours.
   (15-10) The drug for preventing or improving a motor dysfunction according to any one of (15) to (15-9), wherein the tissue plasminogen activator analogue is administered at an amount of 0.3 to 10 mg/kg.
   (15-11) The drug for preventing or improving a motor dysfunction according to (15), wherein the drug for preventing or improving a motor dysfunction is a combined drug.
(16) A drug for enhancing the effect of treating ischemic cerebral stroke of a thrombus removing device comprising the compound represented by the formula (I) as defined in (1), or a pharmaceutically acceptable salt thereof, or a solvate thereof as an active ingredient.
(17) A drug for enhancing the effect of preventing or improving a motor dysfunction caused by ischemic cerebral stroke of a thrombus removing device, comprising the compound represented by the formula (I) as defined in (1), or a pharmaceutically acceptable salt thereof, or a solvate thereof as an active ingredient.
(18) A method of alleviating bleeding tendency caused by a thrombus removing means, comprising administering the compound represented by the formula (I) as defined in (1), or a pharmaceutically acceptable salt thereof, or a solvate thereof.
(19) The method of alleviating bleeding tendency according to (18), wherein the thrombus removing means is a tissue plasminogen activator analogue and/or a thrombus removing device.
(19') A method of alleviating bleeding tendency caused by a tissue plasminogen activator analogue, comprising administering the compound represented by the formula (I) as defined in (1), or a pharmaceutically acceptable salt thereof, or a solvate thereof.
(20) A method of treating ischemic cerebral stroke, comprising combining the compound represented by the formula (I) as defined in (1), or a pharmaceutically acceptable salt thereof, or a solvate thereof and a thrombus removing device.
(21) The method of treating ischemic cerebral stroke according to (20), wherein the thrombus removing device is a tissue plasminogen activator analogue and/or a thrombus removing device.
(21') A method of treating ischemic cerebral stroke, comprising combining the compound represented by the formula (I) as defined in (1), or a pharmaceutically acceptable salt thereof, or a solvate thereof and a tissue plasminogen activator analogue.
(22) The method of treating ischemic cerebral stroke according to any one of (20) to (21'), wherein administration of the thrombus removing means is initiated immediately after, to within 20 hours after ischemic cerebral stroke. (22') The method of treating ischemic cerebral stroke according to any one of (20) to (21'), wherein administration of the tissue plasminogen activator analogue is initiated immediately after, to within 20 hours after ischemic cerebral stroke.
(23) The method of treating ischemic cerebral stroke according to any one of (20) to (22'), wherein administration of the compound represented by the formula (I) as defined in (1), or a pharmaceutically acceptable salt thereof, or a solvate thereof is initiated immediately after, to within 20 hours after ischemic cerebral stroke onset.
(24) A method of treating ischemic cerebral stroke according to any one of (20) to (23), wherein application of the thrombus removing means is initiated during administration of, or within 5 hours after completion of administration of the compound represented by the formula (I) as defined in (1), or a pharmaceutically acceptable salt thereof, or a solvate thereof.
(24') The method of treating ischemic cerebral stroke according to (20) to (23), wherein administration of the tissue plasminogen activator analogue is initiated during administration of, or within 5 hours after completion of administration of the compound represented by the formula (I) as defined in (1), or a pharmaceutically acceptable salt thereof, or a solvate thereof.
(25) The method of treating ischemic cerebral stroke according to any one of (20) to (24'), wherein the compound represented by the formula (I) as defined in (1), or a pharmaceutically acceptable salt thereof, or a solvate thereof is administered by administration route selected from the group consisting of intravenous injection, arterial injection, subcutaneous injection, intracerebral administration and intraspinal administration.
(26) The method of treating ischemic cerebral stroke according to any one of (20) to (25), wherein the compound represented by the formula (I) as defined in (1), or a pharmaceutically acceptable salt thereof, or a solvate thereof is administered by drip infusion.
(27) The method of treating ischemic cerebral stroke according to any one of (20) to (26), wherein the compound represented by the formula (I) as defined in (1), or a pharmaceutically acceptable salt thereof, or a solvate thereof is administered at an amount of 1 to 500 mg/kg.
(28) The method of treating ischemic cerebral stroke according to any one of (20) to (27), wherein the tissue plasminogen activator analogue is administered by administration route selected from the group consisting of intravenous injection, arterial injection, subcutaneous injection, intracerebral administration and intraspinal administration.
(29) The method of treating ischemic cerebral stroke according to any one of (20) to (28), wherein 5% to 20% of a total administration amount of the tissue plasminogen activator analogue is rapidly administered and, thereafter, a remaining amount is administered by drip infusion over 20 minutes to 3 hours.
(30) The method of treating ischemic cerebral stroke according to any one of (20) to (29), wherein the tissue plasminogen activator analogue is administered at an amount of 0.3 to 10 mg/kg.
(31) The method of treating ischemic cerebral stroke according to any one of (20) to (30), wherein the remedy for ischemic cerebral stroke is a combined drug of the compound represented by the formula (I) as defined in (1), or a pharmaceutically acceptable salt thereof, or a solvate thereof, and a tissue plasminogen activator analogue.
(32) The method of preventing or improving a motor dysfunction caused by ischemic cerebral stroke, comprising combining the compound represented by the formula (I) as defined in (1), or a pharmaceutically acceptable salt thereof, or a solvate thereof and a thrombus removing means.
(32') A method of preventing or improving a motor dysfunction caused by ischemic cerebral stroke, comprising combining the compound represented by the formula (I) as defined in (1), or a pharmaceutically acceptable salt thereof, or a solvate thereof and a tissue plasminogen activator analogue.
(33) Use of the compound represented by the formula (I) as defined in (1), or a pharmaceutically acceptable salt thereof, or a solvate thereof for producing a drug for alleviating bleeding tendency caused by a thrombus removing means.
(34) The use according to (33), wherein the thrombus removing means is a tissue plasminogen activator analogue and/or a thrombus removing device. (34') Use of the compound represented by the formula (I) as defined in (1), or a pharmaceutically acceptable salt thereof, or a solvate thereof for producing a drug for alleviating bleeding tendency caused by a tissue plasminogen activator analogue.
(35) Use of the compound represented by the formula (I) as defined in (1), or a pharmaceutically acceptable salt thereof, or a solvate thereof and a tissue plasminogen activator analogue for producing a drug for treating ischemic cerebral stroke.
(36) The use according to (35), wherein administration of the tissue plasminogen activator analogue is initiated immediately after, to within 20 hours after ischemic cerebral stroke onset.
(37) The use according to (35) or (36), wherein administration of the compound represented by the formula (I) as defined in (1), or a pharmaceutically acceptable salt thereof, or a solvate thereof is initiated immediately after, to within 20 hours after ischemic cerebral stroke.
(38) The use according to any one of (35) to (37), wherein administration of the tissue plasminogen activator analogue is initiated during administration of, or within 5 hours after completion of administration of the compound represented by the formula (I) as defined in (1), or a pharmaceutically acceptable salt thereof, or a solvate thereof.
(39) The use according to any one of (35) to (38), wherein the compound represented by the formula (I) as defined in (1), or a pharmaceutically acceptable salt thereof, or a solvate thereof is administered by administration route selected from the group consisting of intravenous injection, arterial injection, subcutaneous injection, intracerebral administration and intraspinal administration.
(40) The use according to any one of (35) to (39), wherein the compound represented by the formula (I) as defined in (1), or a pharmaceutically acceptable salt thereof, or a solvate thereof is administered by drip infusion.
(41) The use according to any one of (35) to (40), wherein the compound represented by the formula (I) as defined in (1), or a pharmaceutically acceptable salt thereof, or a solvate thereof is administered at an amount of 1 to 500 mg/kg.
(42) The use according to any one of (35) to (41), wherein the tissue plasminogen activator analogue is administered by administration route selected from the group consisting of intravenous injection, arterial injection, subcutaneous injection, intracerebral administration and intraspinal administration.
(43) The use according to any one of (35) to (42), wherein 5% to 20% of a total administration amount of the tissue plasminogen activator analogue is rapidly administered and, thereafter, a remaining amount is administered by drip infusion over 20 minutes to 3 hours.
(44) The use according to any one of (35) to (43), wherein the tissue plasminogen activator analogue is administered at an amount of 0.3 to 10 mg/kg.
(45) The use according to any one of (35) to (44), wherein the remedy for ischemic cerebral stroke is a combined drug.
(46) Use of the compound represented by the formula (I) as defined in (1), or a pharmaceutically acceptable salt thereof, or a solvate thereof and a tissue plasminogen activator analogue for producing a drug for preventing or improving a motion functional disorder caused by ischemic cerebral stroke.
(47) Use of the compound represented by the formula (I) as defined in (1), or a pharmaceutically acceptable salt thereof, or a solvate thereof for producing a drug for enhancing the effect of treating ischemic cerebral stroke of a thrombus removing device.
(48) Use of the compound represented by the formula (I) as defined in (1), or a pharmaceutically acceptable salt thereof, or a solvate thereof for producing a drug for enhancing the effect of preventing or improving a motor dysfunction caused by ischemic cerebral stroke of a thrombus removing device.
(49) A combination of the compound represented by the formula (I) as defined in (1), or a pharmaceutically acceptable salt thereof, or a solvate thereof and a thrombus removing means for use in treating ischemic cerebral stroke.
(50) The combination according to (49), wherein the thrombus removing means is a tissue plasminogen activator analogue or a thrombus removing device.
(51) A combination of the compound represented by the formula (I) as defined in (1), or a pharmaceutically acceptable salt thereof, or a solvate thereof and a thrombus removing means for preventing or improving a motor dysfunction caused by ischemic cerebral stroke.

### [Effect of the invention]

The present invention is useful as a remedy for ischemic cerebral stroke, a preventive or a remedy for a motor dysfunction accompanying therewith, and a drug for improving bleeding tendency caused by a thrombus removing means.

### [Brief explanation of the drawings]

Fig. 1 is a view showing an affected side intracerebral hemorrhage area of a control group, compound (I-1) alone administration group, a rtPA alone administration group, a compound (I-1) and a rtPA joint use group.
Fig. 2 is a view showing the result of foot fault test of a control group, a compound (I-1) alone administration group, a rtPA alone administration group, a compound (I-1) and a rtPA joint use group.
Fig. 3 is a view showing the result of an adhesive removed test of a control group, a compound (I-1) alone administration group, a rtPA alone administration group, a compound (I-1) and a rtPA joint use group.
Fig. 4 is a view showing decrease in a weight of a control group (A group), a compound (I-1) alone administration group (B group), a thrombus removing device alone application group (C group), a compound (I-1) and a thrombus removing device joint use group (D group).
Fig. 5 is a view showing an affected side intracerebral hemorrhage area of a control group (A group), a compound (I-1) alone administration group (B group), a thrombus removing device alone application group (C group), and a compound (I-1) and thrombus removing device joint use group (D group).
Fig. 6 is a view showing the result of foot fault test of a control group (A group), a compound (I-1) alone administration group (B group), a thrombus removing device alone application group (C group), and a compound (I-1) and thrombus removing device joint use group (D group).
Fig. 7 is a group showing a cerebral vascular reperfusion arearate of a control group (A group), a compound (I-1) alone administration group (B group), a thrombus removing device alone application group (C group), and a compound (I-1) and thrombus removing device join use group (D group).

### [Best mode for carrying out the invention]

In the present description, the compound represented by the formula (I) is preferably a compound in which R¹ is hydrogen, and R² is -COCH=CHCOOH in the formula (I), a pharmaceutically acceptable salt thereof or a solvent thereof, further preferably a disodium salt of a compound in which R¹ is hydrogen, and R² is -COCH=CHCOOH (hereinafter referred to as compound (I-1)).

The "metabolic ester residue" means an ester residue which can be hydrolyzed in a living body to produce biologically active carboxylic acid.

Examples of the metabolic ester residue include alkyl having a carbon number of 1 to 6 such as methyl, ethyl, and t-butyl; aryl; 1-(acyloxy) lower alkyl such as pivaloyloxymethyl, acetoxymethyl, 1-acetoxyethyl and the like; 1-(lower alkyloxycarbonyloxy) lower alkyl such as 1-(ethoxycarbonyloxy) ethyl, 1-(isopropoxycarbonyloxy) ethyl and the like; (5-methyl-1,3-dioxolen-4-yl) methyl and the like.

The "lower alkyl" means straight or branched alkyl having a carbon number of 1 to 6, and examples include methyl, ethyl, propyl, t-butyl, pentyl, hexyl and the like. A lower alkyl part of the "lower alkyl oxy" is like the "lower alkyl".

The "lower alkylene" includes a divalent carbon chain having a carbon number of 1 to 6, preferably alkylene of a carbon number 1 to 3, more preferably alkylene of carbon number of 1 or 2.

The "lower alkenylene" includes a straight or branched divalent carbon chain of a carbon number of 2 to 6 having a double bond at an arbitrary position. Preferably a carbon number is 2 to 4, more preferably a carbon number is 2 or 3. Examples include vinylene, propenylene, butenylene, butadienylene, methylpropenylene, pentenylene and hexenylene, preferabely vinylene.

The "aryl" includes phenyl and naphthyl and the like.

The "acyl" includes aliphatic acyl and aroyl having a carbon number of 1 to 7. Examples include formyl, acetyl, propionyl, butyryl, isobutyryl, valeryl, pivaloyl, hexanoyl, acryloyl, propioloyl, methacryloyl, crotonoyl and benzoyl.

An acyl part of the "acyloxy" is like the "acyl".

The compound (I) includes also a pharmaceutically acceptable salt thereof, and examples include salts of mineral acids such as hydrochloric acid, sulfuric acid, nitric acid, phosphoric acid, hydrofluoric acid, hydrogen bromide and the like; salts of organic acids such as formic acid, acetic acid, tartaric arid, lactic acid, citric acid, fumaric acid, maleic acid, succinic acid, methanesulfonic acid, ethanesulfonic acid, benzenesulfonic acid, toluenesulfonic acid, naphthalenesulfonic acid, camphorsulfonic acid and the like; salts of organic bases such as ammonium, trimethylammonium, triethylammonium and the like; salts of alkali metals such as sodium, potassium and the like, and salts of alkaline earth metals such as calcium, magnesium and the like. Preferable is a sodium salt, specifically a disodium salt or a trisodium salt.

The compound (I) includes a solvate thereof, and one molecule of the compound (I) may be coordinated with an arbitrary number of solvent molecules. The solvent is preferably water.

In the present invention, the "tissue plasminogen activator analogue" or the "tPA analogue" includes any of a natural type tissue plasminogen activator (natural-type tPA, GenBank Accession No. AA034406), a gene recombinant tissue plasminogen activator (hereinafter, rtPA), a modified type tissue plasminogen activator (hereinafter modified-type tPAT) and animal plasminogen activating factor (blood-sucking bat plasminogen activating factor etc.).

And, rtPA is tPA produced by culturing a host cell other than a human cell transformed with a DNA encoding a human tissue plasminogen activating factor (GenBank Accession No. AY221101) at the condition under which the DNA can be expressed. And, rtPA can be prepared, for example, by the following method using the method described in Molecular Cloning: A Laboratory Manual, Second Edition (1989) (Cold Spring Harbor Laboratory Press), Current Protocols in Molecular Biology (1994) (Wiley-Interscience).

A DNA encoding tPA (human natural type) is obtained by making a cDNA library by a conventional method from a human brain, heart, skeletal muscle, spleen, kidney, liver, small intestine, placenta, a human normal cell derived from these tissues, and a human umbilical venous endothelial cell. In addition, also by performing PCR using oligonucleotides as a sense primer and an antisense primer, and employing, as a template, a cDNA prepared from a mRNA of a cell expressing a mRNA complementary with these DNAs, an objective DNA encoding tPA can be prepared.

Further, the DNA can be prepared by chemically synthesizing a DNA encoding tPA based on an amino acid sequence. Chemical synthesis of a DNA can be performed using a DNA synthesizer manufactured by Shimadzu Corporation utilizing a thiophosphite method, a DNA synthesizer model 392 manufactured by Perkin Elmer utilizing a phosphoamidite method, or the like.

By inserting a DNA of tPA obtained by the above method downstream of a promoter of a suitable expression vector, a recombinant DNA (expression plasmid) is made. By introducing the expression plasmid into a host cell suitable for the expression vector, a transformant producing tPA can be obtained.

As the host cell, a cell which can express an objective gene such as a prokaryotic cell, yeast, an animal cell, a plant cell, an insect cell etc. As the expression vector, an expression vector which can self-replicate in the host cell, or can be incorporated into a chromosome, and contains a promoter at a position suitable for transcription of a gene encoding tPA is used. Examples include an established cell strain of Chinese hamster ovary (CHO cell), mouse C127 cell strain, mouse L cell, mouse fibroblast, mouse NIH3T3 cell, mouse myeloma cell line, COS cell, Hela cell, BHK cell, Bowes cell, human liver-containing cell line (HepG2 etc.), human large intestine fibroblast, human lung-derived diploid fibroblast, blood-sucking bat salivary gland and the like. Preferable is a CHO cell strain.

The modified type tPA includes tPA in which one to a few kinds of sugars in a sugar chain of natural-type tPA are changed, tPA in which one to a few amino acids in natural-type tPA or rtPA are modified with a sugar chain, and tPA in which one to a few amino acids in natural-type tPA or rtPA are substituted with different amino acids modified with a sugar chain.

Herein, a sugar chain is a compound formed by linking one or more unit sugars (monosaccharide or a derivative thereof). Unit sugars are linked by dehydration condensation by a glycoside bond. Examples include monosaccharides such as glucose, galactose, mannose, fucose, glucosamine, N-acetylglucosamine, N-acetylgalactosamine, sialic acid and the like, oligosaccharides such as lactose, maltose, fructose and the like, and a complex and a derivative thereof, degraded polysaccharides, sugar chains degraded or derivatized from complex biological molecules such as glucoprotein, proteoglycan, glycosaminoglycan, and glucolipid, and the like.

The modified type tPA can be appropriately produced by the know method. Examples include a method of changing a kind and a molecular weight of a sugar chain by changing a sugar composition or a sugar concentration in a medium upon preparation of rtPA, a method of changing a sugar chain structure using a cell with glycosyltransferase gene introduced therein, a method of controlling transfer of a galactose residue by adding a sugar in a medium, and a method of introducing a sugar chain into natural-type tPA or rtPA using transglutaminase or the like.

Examples of the preferable tPA analogue used in the present invention include the known tPA analogues which are sold or developed as a general name such as alteplase, amediplase, monteplase, desmoteplase, tenecteplase, reteplase, thisokinase, and pamiteplase and the like.

One aspect of the present invention is a remedy for ischemic cerebral stroke comprising a combination of the compound (I) and a tPA analogue.

The remedy for ischemic cerebral stroke of the present invention can treat ischemic cerebral stroke, and prevent or alleviate various symptoms caused thereby (motor dysfunction and various nerve functional disorders etc.). Ischemic cerebral stroke includes cerebral embolism, cerebral thrombus (lacunar infarction, atherothrombotic infarction etc.), cerebral infarction, transient cerebral ischemia attack, RIND (reversible ischemic neurological deficit), disseminated intravascular coagulation syndrome, and secondary ischemic cerebral stroke developed after cerebral hemorrhage or subarachnoidal hemorrhage. Examples of the symptom caused by ischemic cerebral stroke include motor dysfunction, cognition disorder, sense disorder, language disorder, memory disorder, emotional disorder, thinking ability disorder, dizziness, nausea, vomiting and the like.

When the remedy for ischemic cerebral stroke of the present invention is administered, both of the compound (I) and the tPA analogue can be safely administered by any of oral and parenteral methods, preferably by parenteral administration. For parenteral administration, any dosage form which is usually used such as intravenous injection, arterial injection, subcutaneous injection, intracerebral administration, intraspinal administration, suppository, transdermal absorbing agent, inhalant and the like can be suitably administered, and particularly, intravenous injection or arterial injection (e.g. drip infusion intravenous injection, local administration to a thrombus part using catheter etc.), intracerebral administration, and intraspinal administration are preferable. For oral administration, a form such as powders, granules, tablets, capsules, pills, solutions and the like can be administered. The remedy for ischemic cerebral stroke of the present invention can be prepared by mixing an effective amount of an active ingredient with various pharmaceutical additives such as excipients, binders, wetting agents, disintegrating agents, lubricants, diluents, perfumes and the like suitable for final dosage forms, if necessary. When prepared as injectables, solubilizers, suspending agents, emulsifiers, stabilizers, preservatives, isotonics and the like which are used as an additive for injectables may be appropriately added.

It is desirable that a dose of the compound (I) and the tPA analogue as the remedy for ischemic cerebral stroke is set considering an age, and a weight of a patient, an administration route, a degree of symptom and the like. Usually, the compound (I) may be administered at 1 to 500 mg/kg, preferably 1 mg to 350 mg/kg, more preferably 10 mg to 250 mg/kg. The tPA analogue may be administered at 0.05 mg to 10 mg/kg (about 30000 to 5800000 international unit (IU)), preferably 0.1 mg to 5 mg/kg (about 60000 to 2900000 international unit(IU)), more preferably 0.3 mg to 3 mg/kg (about 170000 to 1700000 international unit(IU)), more preferably 0.5 to 1 mg/kg (about 290000 to 580000 international unit (IU)).

Administration of the compound (I) may be initiated immediately after, to within about 20 hours after ischemic cerebral stroke onset, preferably within about 10 hours, more preferably within 6 hours, more preferably within 3 hours, most preferably within 1 hour.

The administration method is not particularly limited, but includes:
1) a method of continuously administering the dose by drip infusion administration or the like over about 30 minutes to 96 hours, preferably about 1 hour to 72 hours, more preferably about 3 hours to about 48 hours,
2) a method of rapidly administering about 5% to 30%, preferably about 10% to 20% of the dose within the above administration time by intravenous injection, arterial injection, subcutaneous injection, intracerebral administration or intraspinal administration and, thereafter, continuously administering a remaining amount by drip infusion administration or the like over about 30 minutes to 96 hours, preferably about 1 hour to 72 hours, more preferably about 3 hours to about 48 hours,
3) a method of repetitively intermittently administering 1 unit to 5 units per day, preferably 1 unit to 3 units per day, letting administration of the dose for about 30 minutes to about 10 hours, preferably about 1 hour to about 5 hours and, thereafter, cessation of the drug for about 1 hour to 15 hours, preferably about 3 hours to about 10 hours to be one unit.

Rapid administration and continuous administration may be performed continuously, or may be performed at an interval of about 1 hour to 15 hours, preferably about 3 hours to 10 hours.

Administration of the tPA analogue may be initiated immediately after, to within about 20 hours after ischemic cerebral stroke onset, preferably within about 10 hours, more preferably within 7 hours, more preferably within 5 hours, more preferably 3 hours.

The administration method is not particularly limited, but examples include a method of continuously administering the dose by drip infusion administration or the like over about 30 minutes to 96 hours, preferably about 1 hour to 72 hours, more preferably about 3 hours to abut 48 hours, a method of rapidly administering about 5% to 30%, preferably about 10% to 20% of the dose by intravenous injection, arterial injection, subcutaneous injection or the like and, thereafter, continuously administering a remaining amount by drip infusion administration or the like over about 20 minutes to 12 hours, preferably about 30 minutes to 5 hours, more preferably about 30 minutes to about 2 hours, a method of rapidly administering the dose by intravenous injection, arterial injection, subcutaneous injection or the like by dividing the dose into one time to a few times, and the like. Raid administration and continuous administration may be continuously performed, or may be performed at an interval of about 1 hour to 15 hours.

An interval between administration of the compound (I) and administration of the tPA analogue is not particularly limited. Therefore, any of them (e.g. compound (I)) may be first initiated to be administered continuously, or within about 15 hours, preferably within about 10 hours, preferably within about 5 hours, preferably within about 3 hours, more preferably within about 2 hours, the other may be administered, or both compounds may be administered simultaneously.

In addition, as the compound (I) and the tPA analogue, those that were separately prepared may be used, or a form of a kit or a combined preparation may be used.

In addition to the compound (I) and the tPA analogue, other anti-thrombus drug may be further used together. The anti-thrombus drug includes a blood coagulation arresting drug and a thrombolytic drug, preferably a thrombolytic drug. Specifically, examples include the tPA analogue, urokinase, streptokinase, sarpogrelate hydrochloride, ozagrel sodium, nafamostat mesylate, eptifibatide, clopidogrel sulfate, cilostazol, batroxobin, aspirin, argatroban, anistreplase, ancrod, alfimeprase, prasugrel and the like.

As an example of administration schedule of the compound (I) and the tPA analogue, the following methods are possible.

### 1) Schedule example 1

Time of compound (I) administration initiation: immediately after, to within about 20 hours after ischemic cerebral stroke onset
Dose of compound (I): 1 mg to 500 mg/kg
Method of administration of compound (I): continuous administration for about 30 minutes 96 hours
Time of tPA analogue administration initiation: within 5 hours after compound (I) administration initiation
Dose of tPA analogue: 0.05 mg to 10 mg/kg
Method of administration of tPA analogue: rapid administration of about 5% to 30% of a dose, and continuous administration of a remaining amount over 3 minutes to 5 hours

### 2) Schedule example 2

Time of compound (I) administration initiation: immediately after, to within about 10 hours after ischemic cerebral stroke onset
Dose of compound (I): 10 mg to 400 mg/kg
Method of administration of compound (I): continuous administration for about 1 hour to 72 hours
Time of tPA analogue administration initiation: within 3 hours after compound (I) administration initiation
Dose of tPA analogue: 0.1 mg to 5 mg/kg
Method of administration of tPA analogue: rapid administration of about 10% to about 20% of a dose, and continuous administration of a remaining amount over about 30 minutes to 2 hours

### 3) Schedule example 3

Time of compound (I) administration initiation: immediately after, to within about 6 hours after ischemic cerebral stroke onset
Dose of compound (I): 20 mg to 400 mg/kg
Method of administration of compound (I): continuous administration for 1 hour to 72 hours
Time of tPA analogue administration initiation: within 2 hours after compound (I) administration initiation
Dose of tPA analogue: 0.3 mg to 3 mg/kg
Method of administration of tPA analogue: rapid administration of about 10% to 20% of a dose, and continuous administration of a remaining amount over about 30 minutes to 2 hours

### 3) Schedule example 4

Time of compound (I) administration initiation: immediately after, to within 3 hours after ischemic cerebral stroke onset
Dose of compound (I): 30 mg to 300 mg/kg
Method of administration of compound (I): continuous administration for about 1 hour to 72 hours
Time of tPA analogue administration initiation: within 2 hours after compound (I) administration initiation
Dose of tPA analogue: 0.3 mg to 3 mg/kg
Method of administration of tPA analogue: rapid administration of about 10% to 20% of a dose, and continuous administration of a remaining amount over about 30 minutes to 2 hours

### 5) Schedule example 5

Time of compound (I) administration initiation: immediately after, to within about 6 hours after ischemic cerebral stroke onset
Dose of compound (I): 20 mg to 400 mg/kg
Method of administration of compound (I): rapid administration of about 10% to 20% of a dose, and continuous administration of a remaining amount over about 1 hour to 72 hours
Time of tPA analogue administration initiation: within 5 hours after initiation of initial administration of compound (I)
Dose of tPA analogue: 0.3 mg to 3 mg/kg
Method of administration of tPA analogue: rapid administration of about 10% to 20% of a dose, and continuous administration of a remaining amount over about 30 minutes to 2 hours

### 6) Schedule example 6

Time of compound (I) administration initiation: immediately after, to within about 6 hours after ischemic cerebral stroke onset
Dose of compound (I): 20 mg to 400 mg/kg
Method of administration of compound (I): continuous administration for about 1 hour to 72 hours
Time of tPA analogue administration initiation: simultaneously with compound (I)
Dose of tPA analogue: 0.3 mg to 3 mg/kg
Method of administration of tPA analogue: rapid administration of about 10% to 20% of a dose, and continuous administration of a remaining amount over about 30 minutes to 2 hours

### 7) Schedule example 7

Time of compound (I) administration initiation: immediately after, to within about 20 hours after ischemic cerebral stroke onset
Dose of compound (I): 1 mg to 500 mg/kg
Method of administration of compound (I): repetitive intermittent administration of 1 unit to 3 units per day, letting administration for about 30 minutes to about 10 hours and, thereafter, cessation of the drug for about 1 hour to 15 hours to be one unit
Time of tPA analogue administration initiation: within 5 hours after initiation of initial administration of compound (I)
Dose of tPA analogue: 0.3 mg to 3 mg/kg
Method of administration of tPA analogue: rapid administration of about 10% to 20% of a dose, and continuous administration of a remaining amount over about 30 minutes to 2 hours

Another aspect of the present invention is a drug for enhancing the effect of treating cerebral stroke of a tPA analogue, or a drug for enhancing the effect of preventing or improving a motor dysfunction caused by ischemic cerebral stroke of a tPA analogue, comprising the compound (I) as an active ingredient. A dose, and an administration time of the compound (I), and a dose, and an administration time of a tPA analogue as an enhancing drug are not particularly limited. For example, they are the same as those of the case of use as the remedy for ischemic cerebral stroke.

Another aspect of the present invention is a drug for alleviating bleeding tendency caused by a thrombus removing means comprising the compound (I) as an active ingredient.

The "alleviation of bleeding tendency caused by a thrombus removing means" indicates significant decrease in an area of intracerebral bleeding generated by application of a thrombus removing means.

The "thrombus removing means" includes all methods of removing thrombus generated by ischemic cerebral stroke. Examples include a chemical thrombus removing means such as thrombus lysing with the tPA analogue, a mechanical or physical thrombus removing means with a thrombus removing device, and joint use of them.

The "thrombus removing device" is not particularly limited as far as it is a device for thrombus physically, chemically or mechanically. Examples of the method of removing a thrombus include a method of using a physiologically saline ejection suction (suction-creating salin jet), a method of using a laser energy, a method of using an ultrasound, a corkscrew method, a method of abstraction using a syringe, and the like. Specific examples include devices such as MERCI (registered trademark) Retrieval System (manufactured by Concentric), Ultrasound Thrombolytic Infusion catheter (manufactured by EKOS).

A dose, an administration time and the like of the compound (I) and the tPA analogue as a drug for improving bleeding tendency caused by the thrombus removing means are not particularly limited. For example, they are the same as those of the case of use as the remedy for ischemic cerebral stroke.

Since thrombus removal with the thrombus removing device is different in an application method depending on each device, operation may be performed according to instructions attached to each device. For example, application may be initiated according to instructions immediately after, to within about 20 hours after ischemic cerebral stroke, preferably within about 10 hours, more preferably within 7 hours, more preferably within 5 hours, more preferably within 3 hours.

An interval between administration of the compound (I) and application of the thrombus removing device is not particularly limited. Therefore, after rapid administration of the compound (I), treatment with the thrombus removing device may be performed, for example, within about 15 hours, preferably within about 10 hours, preferably within about 5 hours, preferably within about 3 hours, more preferably within about 2 hours, or treatment with the thrombus removing device may be performed during continuous administration of the compound (I). Further, after treatment with the thrombus removing device, the compound (I) may be administered within about 15 hours, preferably within about 10 hours, preferably within about 5 hours, preferably within about 3 hours, more preferably within about 2 hours.

It is also possible to use with an anti-thrombus drug depending on a kind of the thrombus removing device used and a morbid of a patient. Therefore, three kinds of the compound (I), the thrombus removing device and the anti-thrombus drug comprising the tPA analogue may be used jointly. The anti-thrombus drug includes a blood coagulation arresting drug and a thrombolytic, preferably a thrombolytic. Specifically, examples include the tPA analogue, urokinase, streptokinase, sarpogrelate hydrochloride, ozagrel sodium, nafamostat mesylate, eptifibatide, clopidogrel sulfate, cilostazol, batroxobin, aspirin, argatroban, anistreplase, ancrod, alfimeprase, prasugrel and the like.

When the thrombus removing device is a type to be used with the anti-thrombus drug jointly, after or before administration of the compound (I), treatment with a device according to a method of using the device, and administration of the anti-thrombus drug may be performed. Alternatively, it is also possible that the compound (I) and the anti-thrombus drug are conveniently administered and, thereafter, treatment with the thrombus removing device is performed.

An administration method and a dose of the compound (I), an application method and an application time of the thrombus removing device, and an administration method and a dose of the anti-thrombus drug to be used jointly are, for example, as follows.

### 8) Schedule example 8

Time of compound (I) administration initiation: immediately after, to within about 20 hours after ischemic cerebral stroke onset
Dose of compound (I): 1 mg to 500 mg/kg
Method of administration of compound (I): continuous administration for about 30 minutes to 96 hours
Time of application initiation of thrombus removing device: within 5 hours after initiation of compound (I) administration

### 9) Schedule example 9

Time of compound (I) administration initiation: immediately after, to within about 6 hours after ischemic cerebral stroke
Dose of compound (I): 20 mg to 400 mg/kg
Method of administration of compound (I): rapid administration of about 10% to 20% of a dose, and continuous administration of a remaining amount over about 1 hour to 72 hours
Time of application initiation of thrombus removing device: within 5 hours after initiation of initial administration of compound (I)

### 10) Schedule example 10

Time of compound (I) administration initiation: immediately after, to within about 6 hours after ischemic cerebral stroke onset
Dose of compound (I): 1 mg to 500 mg/kg
Method of administration of compound (I): continuous administration for about 30 minutes to 96 hours
Time of application initiation of thrombus removing device: within 8 hours after ischemic cerebral stroke onset

### 11) Schedule example 11

Time of compound (I) administration initiation: immediately after, to within about 6 hours after ischemic cerebral stroke onset
Dose of compound (I): 20 mg to 400 mg/kg
Method of administration of compound (I): rapid administration of about 10% to 20% of a dose, and continuous administration of a remaining amount over about 1 hour to 72 hours
Time of application initiation of thrombus removing device: within 8 hours after ischemic cerebral stroke onset

### 12) Schedule example 12

Time of compound (I) administration initiation: immediately after, to within about 6 hours after ischemic cerebral stroke onset
Dose of compound (I): 20 mg to 400 mg/kg
Method of administration of compound (I): continuous administration for about 1 hour to 72 hours
Time of application initiation of thrombus removing device: within 2 hours after administration initiation of compound (I)
Dose of thrombolytic drug: different depending on a kind of a thrombolytic drug, for example, 0.3 mg to 300 mg/kg
Method of administration of thrombolytic drug: administration according to instructions of thrombus removing device

### 13) Schedule example 12

Time of compound (I) administration initiation: immediately after, to within about 6 hours after ischemic cerebral stroke
Dose of compound (I): 20 mg to 400 mg/kg
Method of administration of compound (I): continuous administration for about 1 hour to 72 hours
Time of administration initiation of tPA analogue: within 5 hours after administration initiation of compound (I)
Dose of tPA analogue: 0.3 mg to 300 mg/kg
Method of administration of tPA: rapid administration of about 10% to 20% of a dose, and continuous administration of a remaining amount over about 30 minuets to 2 hours
Time of application initiation of thrombus removing device: within 5 hours after administration initiation of compound (I)

Another aspect of the present invention is a drug for preventing or improving a motion functional disorder caused by ischemic cerebral stroke comprising the compound (I) and the tPA analogue as an active ingredient.

A dose and an administration time of the compound (I) and the tPA analogue as a drug for preventing or improving a motion functional disorder caused by ischemic cerebral stroke, as well as an application method and an application time of the thrombus removing device are not particularly limited. For example, they are the same as those of the case of use as the remedy for ischemic cerebral stroke.

Another aspect of the present invention is a drug for enhancing the effect of treating ischemic cerebral stroke of the thrombus removing device comprising the compound (I) as an active ingredient.

A dose and an administration time of the compound (I) as a drug for enhancing the effect of treating ischemic cerebral stroke of the thrombus removing device are not particularly limited. For example, they are the same as those of the case of use as a drug of alleviating bleeding tendency.

Since an application method, and an application time of the thrombus removing device are different depending on each device, operation may be performed according to instructions attached to each device. For example, application may be initiated according to instructions immediately after, to within about 20 hours after ischemic cerebral stroke onset, preferably within about 10 hours, more preferably within 8 hours, more preferably within 5 hours, more preferably within 3 hours.

Another aspect of the present invention is a drug for enhancing the effect of preventing or improving a motor dysfunction caused by ischemic cerebral stroke of the thrombus removing device comprising the compound (I) as an active ingredient.

A dose and an administration time of the compound (I) as a drug for enhancing the effect of preventing or improving a motor dysfunction caused by ischemic cerebral stroke are not particularly limited. For example, they are the same as those of the case of use as the drug for alleviating bleeding tendency.

Since an application method and an application time of the thrombus removing device are different depending on each device, operation may be performed according to instructions attached to each device. For example, application may be initiated according to instructions immediately after, to within about 20 hours after ischemic cerebral stroke onset, preferably within about 10 hours, more preferably within 8 hours, more preferably within 5 hours, more preferably within 3 hours.

Another aspect of the present invention is a drug for prolonging a time window (time rage in which treatment is possible from onset) of the thrombus removing means comprising the compound (I) as an active ingredient. Each thrombus removing means is reduced in the treatment effect as an application time of the thrombus removing means from ischemic cerebral stroke onset becomes longer, and it is thought that a risk such as bleeding becomes higher. However, by joint use with the compound (I), significant effect is seen in motor function improvement also at the condition under which high effect is not seen by application of the thrombus removing means alone, and the synergistic effect due to joint use can be obtained. In addition, by joint use, a risk such as bleeding is significantly reduced as compared with alone application.

The "time window" is, for example,
"longest time from stroke onset to initiation of application of the thrombus removing means" during which the thrombus removing means can exhibit the significant effect on improvement in motor function or cerebral stroke treatment, or
"longest time from stroke onset to application initiation of the thrombus removing means" under which the thrombus removing means shows the significant effect on improvement in motion function or cerebral stroke treatment, and bleeding is not caused.

The "prolongation of time window" means that time window of the case of joint use of the compound (I) and the thrombus removing means is prolonged 10% or more as compared with time window of the case of application of the thrombus removing means alone. The prolongation includes prolongation of preferably 20% or more, further preferably 30% or more, further preferably 50% or more, further preferably 80% or more, further preferably 100% or more, further preferably 200% or more, further preferably 300% or more, further preferably 400% or more.

A dose and an administration time of the compound (I) and the tPA analogue as a drug for prolonging a time window of the thrombus removing means, as well as a method of application of, an application time of the thrombus removing device, and the like are not particularly limited. For example, they are the same as those of the case of use as the remedy for ischemic cerebral stroke, or a drug for improving bleeding tendency caused by the thrombus removing means.

It is known that the compound (I) exhibits the activity of inhibiting brain edema by sole administration and, it is also possible to treat or prevent cerebral edema by administering the compound (I) alone after joint use of the thrombus removing means.

The compound (I) exhibits the activity of treating ischemic cerebral stroke, the activity of inhibiting development of a cerebral infarction lesion, the activity of preventing or improving a motor dysfuction caused by ischemic cerebral stroke, and the activity of preventing or improving nerve symptom caused by ischemic cerebral stroke also in sole administration, and the synergistic effect on these activities is exhibited by administering a combination of the compound (I) and the thrombus removing means.

As compared with the case of administration of the compound (I) or the tPA analogue alone, each dose can be also reduced, and a remedy for ischemic cerebral stroke with the alleviate side effect can be also obtained.

### Examples

### Test Example 1 Effect of compound (I-1) on therapeutic time window (time range in which treatment is possible from stroke onset) and intracerebral hemorrhage of rtPA in a rat model of embolic stroke.

### (1) Experimental method

A cannulae was dwelled into a right femoral artery and a vein of a rat under anesthesia, and blood clot produced by fresh autologous arterial blood and thrombin was injected into the origin of a middle cerebral artery (MCA) through a catheter inserted through an inverted outer carotid artery to make a embolic stroke model. After 90 minutes from MCA occlusion, an animal exhibiting an ischemic neurological symptom was used in an experiment thereafter. During operation, a body temperature was maintained at 37°C using a temperature retaining device while an intrarectal temperature was continuously measured. A blood pressure was measured using a femoral artery cannulae, and pH, pCO2, and pO2 of arterial blood were monitored continuously. Drug administration was performed using a femoral vein cannulae. Rats were divided into 4 groups (A: control (physiological saline) group, B: recombinant tissue plasminogen activator (rtPA) alone administration group, C: compound (I-1) alone administration group, and D: compound (I-1) and rtPA concomitant use group), and an experiment was performed.

The compound (I-1)(3 mg/kg/h) was continuously administered from 2 hours to 74 hours after MCA occlusion, and 1 mg/kg of rtPA was rapidly administered 4 hours after MCA occlusion and, thereafter, 9 mg/kg was continuously administered over 30 minutes. In the C or B group, a physiological saline was administered in place of rtPA or the compound (I-1), respectively, and in the A group, a physiological saline was administered in place of rtPA and the compound (I-1) by the similar operation. An intravenous injection rate was 2 ml/kg/h.

A motor function was measured immediately before MCA occlusion and 7 days after occlusion, respectively. A motor dysfuction was studied using an Adhesive removed test and a Foot fault test. In the case of the Adhesive removed test, a ring of a packing tape was attached to a forefoot, and a time (required time) until it was peeled was used as an index. In addition, in the Foot fault test, after a rat was placed on a metal net, a behavior was recorded in a video tape, and this was quantitated using, as an index, a ratio of slipping a foot from a wire of a metal net (left forefoot slipping time).

Seven days after MCA occlusion, a motor dysfuction was measured, then 10% formalin was infused transcardially anesthesia, a brain was isolated, and a paraffin coronal serial section was made. Further, each section was stained using hamatoxylan and eosin and, as an affected side intracerebral hemorrhage area, an area of a site in which erythrocyte was leaked from a blood vessel in each section was calculated using an image analyzing program.

### (2) Results

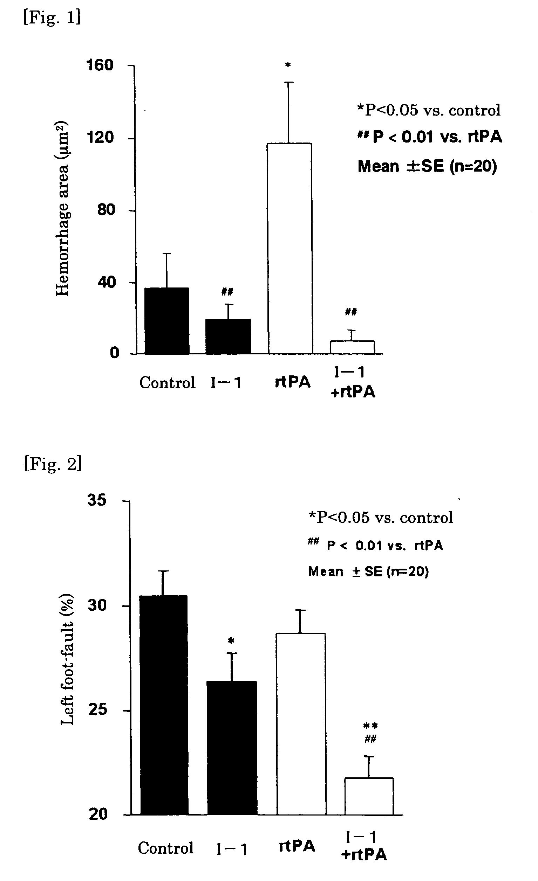

Results are shown in Figs 1 to 3.

From Fig. 1, it is seen that the compound (I-1) has the activity of alleviating bleeding tendency with the tPA analogue.

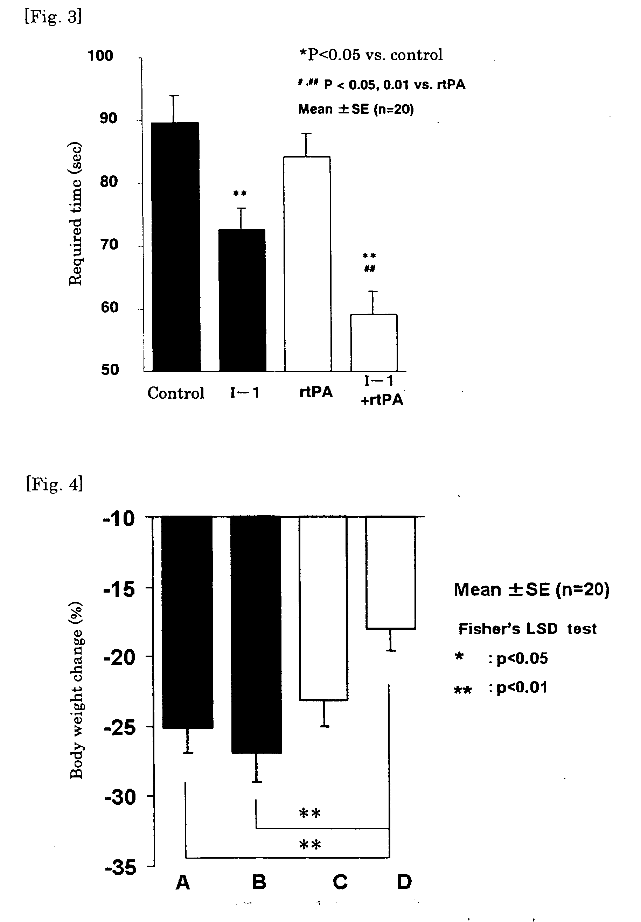

From Fig.2 and Fig.3, it is clear that the compound (I-1) has the activity of improving a motor dysfunction also in alone administration, while concomitant use with the tPA analogue significantly exhibits the activity of improving a motion functional disorder as compared with administration of the compound alone. That is, concomitant administration of the tPA analogue and the compound (I-1) exhibits the high synergistic activity, and exhibits the excellent activity of improving a motor dysfuction.

### Test Example 2 Effect of concomitant treatment with thrombus removing device and compound (I-1)

### 1) Experimental method

Under anesthesia, a cannulae was dwelled in a right femoral artery and a vein of a rat, and a right common carotid artery, an internal carotid artery and an external carotid artery were exposed. A length of 4-0 monofilament nylon suture (18.5 to 19.5mm: determined by animal) was advanced from the ECA into the lumen of the internal carotid artery until it blocked the origin of the middle cerebral artery (MCA). Thirty minutes after occlusion, only a rat exhibiting an ischemic neurological symptom served as an ischemic loading successful example in a test thereafter.

During operation, a body temperature was maintained at 37°C using a temperature retaining device while an intrarectal temperature was continuously measured. In a recanalization group following 2 h MCA occlusion, animal was re-anesthetized and recanaization was achieved by withdrawal of the suture (Withdrawal of the suture corresponds to thrombus removal with a thrombus removing device). In addition, in a one week MCA occlusion group, the suture was placed at the origin of the MCA for 1 week. A blood pressure was measured using a cannulae inserted into a femoral artery. In addition, administration of a drug was performed using a cannulae inserted into a femoral vein.

An experiment was performed by dividing rats into four groups (A: one week MCA occlusion/Vehicle administration group, B: one week MCA occlusion/compound (I-1) administration group, C: recanalization following 2 hours MCA occlusion/Vehicle administration group, D: recanalization following 2 hours MCA occlusion/compound (I-1) administration group).

The compound (I-1) (3 mg/kg/h) or Vehicle (physiological saline) was continuously administered from 1 hour to 73 hours after MCA occlusion using a syringe pump (intravenous infusion rate: 2 mL/kg/h).

A motor dysfunction was studied using a Foot fault test, and evaluated immediately after and 7 days after MCA occlusion. That is, the rat was placed on a metal net, a behavior was recorded in a video tape, and this was quantitated using, as an index, a ratio of slipping a foot from a wire of a metal net.

Seven days after MCA occlusion, a motor function and body weight were measured and, thereafter, isothiocyanate (FITC) dextran was intravenously administered to the rat. After five minutes, a brain was isolated under anesthesia, and a serial paraffin coronal section was made. A fluorescent intensity of each section was measured using an image analyzer, and a rate of cerebral microvessel patency was determined. That is, the number of pixels exhibiting fluorescence over a set threshold was expressed by % relative to the number of pixels of a whole region in a right MCA governing region of each coronal section. An effected side intracerebral hemorrhage area was evaluated by the leakage of erythrocyte from blood vessels in each section and was calculated using an image analyzing program.

### 2) Results

Results are shown in Figs 4 to 7.

From Fig.4, at concomitant use with the thrombus removing device and the compound (I-1) (D group), reduction of body weight loss accompanied with an ischemic stress was observed as compared with other groups, and it indicates that damage on the animal is alleviated. An average weight before MCA occlusion was 288 g in the control group (A group), 290 g in the compound (I-1) alone administration group (B group), 292 g in the thrombus removing device alone application group (C group), and 291 g in the compound (I-1) and the thrombus removing device concomitant use group (D group), respectively, and no significance difference was seen between respective groups.

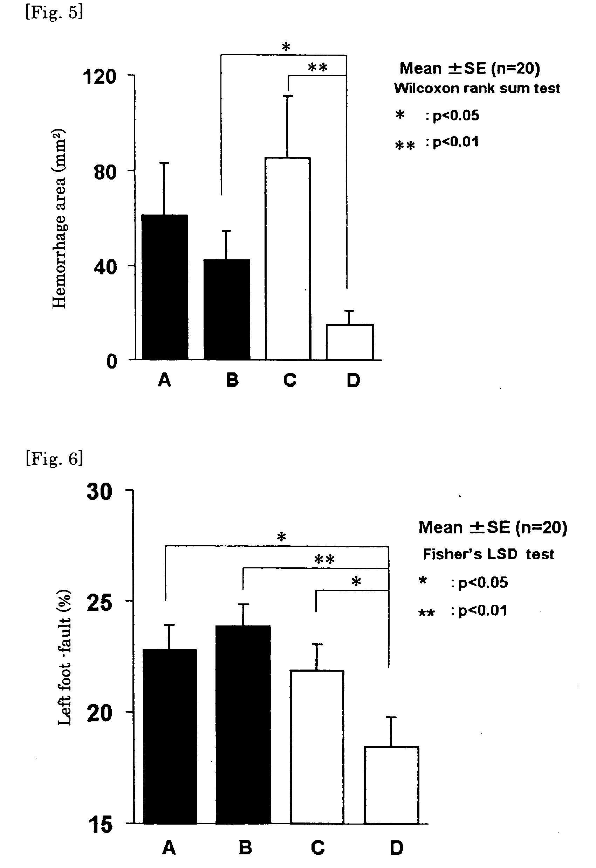

From Fig.5, it is seen that remarkable decrease in the hemorrhage area is observed in the thrombus removing device and the compound (I-1) concomitant use (D group) as compared with other groups.

From Fig.6, a motor dysfuction is remarkably improved in the concomitant use of the thrombus removing device and the compound (I-1) (D group) as compared with the compound (I-1) alone administration (B group), and the thrombus removing device alone application (C group), and it is understood that the synergistic effect due to concomitant use of the compound (I-1) and the thrombus removing device is seen.

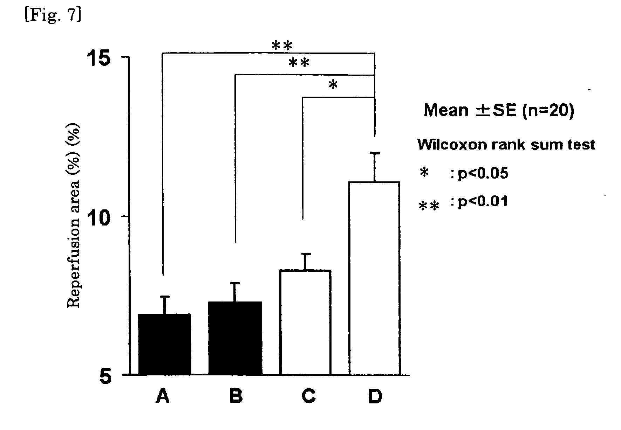

From Fig.7, a rate of cerebral microvessel patency (reperfusion area) in the MCA governing lesion is increased in the concomitant use of the thrombus removing device and the compound (I-1) (D group) as compared with the compound (I-1) alone administration (B group) and the thrombus removing device alone administration (C group), and it is understood that the synergistic effect is seen due to joint use of the compound (I-1) and the thrombus removing device.

### Preparation Example 1 Drug for alleviating bleeding tendency by tPA analogue

- Compound (I-1): 90 g
- D mannitol: 45.0 g
- Water injection: 1500 g

The above ingredients are mixed, and stirred. One mol/l sodium hydroxide is added in portions until a pH becomes 9.5, and water for injection is added to a total amount of 1800 g. The resulting solution is roughly filtered with a 0.45 µm filter, and further sterilization-filtered with a 0.22 µm filter. This filtrate is dispensed at 6.0 g per vial, and lyophilized to obtain the preparation injection.

### Preparation Example 2 Kit treating ischemic cerebral stroke

- Alteplase: 12000000 international unit
- L- arginine: 579-869 mg
- Polysorbate 80: 0.4-3.8 mg

### Phosphoric acid

By combining an alteplase preparation containing the above ingredients, the preparation obtained in Preparation Example 1, and water for injection as a solution for dissolving them, a kit for treating ischemic cerebral stroke is obtained.

### [Industrial applicability]

The present invention is effective in treating ischemic cerebral stroke (cerebral infarction etc.).

## Claims

1. A drug for alleviating bleeding tendency caused by a thrombus removing means comprising, as an active ingredient, a compound represented by formula (I): 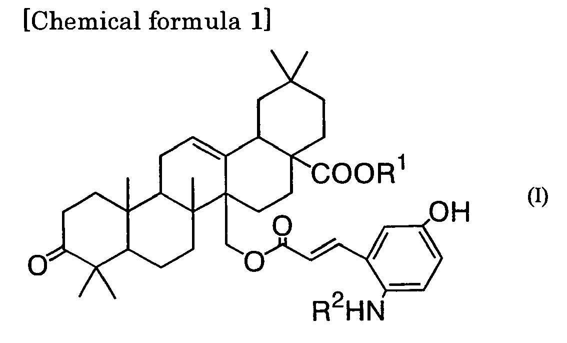 (wherein R¹ represents hydrogen or a metabolic ester residue, R² represents hydrogen or -R³-R⁴ (wherein R³ represents -SO₃-, -CH₂COO-, -COCOO- or -COR⁵COO- (wherein R⁵ represents lower alkylene or lower alkenylene), and R⁴ represents hydrogen or a metabolic ether residue)),
or a pharmaceutically acceptable salt thereof, or a solvate thereof.

2. The drug for alleviating bleeding tendency according to Claim (1), wherein the thrombus removing means is a tissue plasminogen activator analogue and/or a thrombus removing device.

3. A remedy for ischemic cerebral stroke, comprising a combination of the compound represented by the formula (I) as defined in Claim (1), or a pharmaceutically acceptable salt thereof, or a solvate thereof and a tissue plasminogen activator analogue.

4. The remedy for ischemic cerebral stroke according to Claim (3), which is a kit containing a drug containing the compound represented by the formula (I) as defined in Claim (1), or a pharmaceutically acceptable salt thereof, or a solvate thereof, and a drug containing a tissue plasminogen activator analogue.

5. The remedy for ischemic cerebral stroke according to Claim (3) or (4), wherein administration of the tissue plasminogen activator analogue is initiated immediately after, to within 20 hours after ischemic cerebral stroke onset.

6. The remedy for ischemic cerebral stroke according to any one of Claim (3) to (5), wherein administration of the compound represented by the formula (I) as defined in (1), or a pharmaceutically acceptable salt thereof, or a solvate thereof is initiated immediately after, to within 20 hours after ischemic cerebral stroke onset.

7. The remedy for ischemic cerebral stroke according to any one of Claim (3) to (6), wherein administration of the tissue plasminogen activator analogue is initiated during administration of, or within 5 hours after completion of administration of the compound represented by the formula (I) as defined in Claim (1), or a pharmaceutically acceptable salt thereof, or a solvate thereof.

8. The remedy for ischemic cerebral stroke according to any one of Claim (3) to (7), wherein the compound represented by the formula (I) as defined in (1), or a pharmaceutically acceptable salt thereof, or a solvate thereof is administered by administration route selected from the group consisting of intravenous injection, arterial injection, subcutaneous injection, intracerebral administration and intraspinal administration.

9. The remedy for ischemic cerebral stroke according to any one of Claim (3) to (8), wherein the compound represented by the formula (I) as defined in Claim (1), or a pharmaceutically acceptable salt thereof, or a solvate thereof is administered by drip infusion.

10. The remedy for ischemic cerebral stroke according to any one of Claim (3) to (9), wherein the compound represented by the formula (I) as defined in Claim (1), or a pharmaceutically acceptable salt thereof, or a solvate thereof is administered at an amount of 1 to 500 mg/kg.

11. The remedy for ischemic cerebral stroke according to any one of Claim (3) to (10), wherein the tissue plasminogen activator analogue is administered by administration route selected from the group consisting of intravenous injection, arterial injection, subcutaneous injection, intracerebral administration and intraspinal administration.

12. The remedy for ischemic cerebral stroke according to any one of Claim (3) to (11), wherein 5% to 20% of a total administration amount of the tissue plasminogen activator analogue is rapidly administered and, thereafter, a remaining amount of the analogue is administered by drip infusion over 20 minutes to 3 hours.

13. The remedy for ischemic cerebral stroke according to any one of Claim (3) to (12), wherein the tissue plasminogen activator analogue is administered at an amount of 0.3 to 10 mg/kg.

14. The remedy for ischemic cerebral stroke according to Claim (3), wherein the remedy for ischemic cerebral stroke is a combined drug.

15. A drug for preventing or improving a motor dysfunction caused by ischemic cerebral stroke, comprising a combination of the compound represented by the formula (I) as defined in Claim (1), or a pharmaceutically acceptable salt thereof, or a solvate thereof and a tissue plasminogen activator analogue.

16. A drug for enhancing the effect of treating ischemic cerebral stroke of a thrombus removing device comprising the compound represented by the formula (I) as defined in Claim (1), or a pharmaceutically acceptable salt thereof, or a solvate thereof as an active ingredient.

17. A drug for enhancing the effect of preventing or improving a motor dysfunction caused by ischemic cerebral stroke of a thrombus removing device, comprising the compound represented by the formula (I) as defined in Claim (1), or a pharmaceutically acceptable salt thereof, or a solvate thereof as an active ingredient.
